# EUROPEAN PATENT APPLICATION

(11) **EP 2 497 464 A2**
(43) Date of publication of application: **12.09.2012**
(21) Application number: 12156117.9
(22) Date of filing: 20.02.2012
(51) Int. Cl.: A61K 9/16, A61K 9/48, A61K 31/506

(54) **Pharmaceutical composition of imatinibe methanesulphonate and a process for its manufacture**

(30) Priority: 09.03.2011 PL 39416911
(71) Applicant: Adamed SP. Z O.O., 05-152 Czosnów k/Warszawy (PL)
(72) Inventor: Ilczuk, Jerzy Adam, 05-200 Wolomin (PL); Komsta-Leszczyk, Izabela Sylwia, 78-301 Swidwin (PL)
(74) Representative: Sitkowska, Jadwiga

(57) **Abstract**

Pharmaceutical composition of imatinib methanesulfonate has the form of a solid blend and consists of: a) from 99,7 to 96% by weight of granulate of imatinib methanesulfonate, wherein the granulate contains, based on total weight of the granulate, a1) from 81 to 100% by weight of imatinib methanesulfonate in the crystalline form alpha and a2) from 0 to 19% by weight of one or more pharmaceutical excipients; b) from 0,3 to 3% by weight of an external lubricant; and c) from 0 to 1% by weight of an extragranular glidant. The composition is manufactured by dry granulation of imatinib methanesulfonate, optionally with excipients, without using any solvents, followed by blending the resulting granulate with the lubricant, and optionally the glidant. The blend thus prepared is used for filling unit dosage forms.

## Description

The invention relates to a solid pharmaceutical composition of imatinib methanesulfonate, specifically imatinib methanesulfonate in the crystalline alpha form, designed for filling into unit dosage forms, to a process for manufacturing the composition and to unit dosage forms containing it.

Imatinib is the INN (International Non-proprietary Name) of a compound having the systematic chemical name 4-(4-methylpiperazin-1-ylmethyl)-N-[4-methyl-3-[(4-pyridin-3-yl)pyrimidin-2-ylamino]phenyl]benzamide and the following structural formula (I):

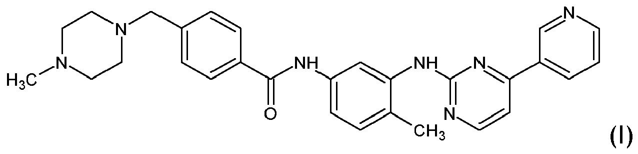

Imatinib is a protein tyrosine kinase inhibitor and is used for the treatment of cancer diseases. Its structure, activity and usefulness in medicine were first disclosed in EP0564409A.

Imatinib as a methanesulfonate salt is the active ingredient of Glivec, a drug used for the treatment of chronic myeloid leukaemia (CML), Ph+ acute lymphoblastic leukaemia (ALL), myelodysplastic or myeloproliferative diseases (MD/MPD), advanced hypereosinophilic syndrome (HES) or chronic eosinophilic leukaemia (CEL), gastrointestinal stromal tumours (GIST), and dermatofibrosarcoma protuberans (DFSP).

Imatinib methanesulfonate (mesylate) exhibits polymorphism of crystalline forms. In WO 99/03854 two crystalline forms, alpha and beta, of imatinib mesylate were disclosed, characterized among others by characteristic X-ray powder diffraction patterns. Further crystal forms, I and II, were described in WO 05/095379, form alpha2 - in WO 05/077933, form H1 - in WO 04/106326, forms delta and epsilon - in WO 2007/023182. The forms mentioned above, particularly those which are metastable such as the alpha form of imatinib methanesulfonate, may convert into a thermodynamically more stable form, for example while treated with certain solvents.

Crystalline alpha form of imatinib methanesulfonate was described as exhibiting slightly higher hygroscopicity than beta form under conditions of high humidity. Alpha form has a characteristic powder diffraction pattern shown in Fig. 1 of WO 99/03854.The prior art describes also several pharmaceutical compositions comprising imatinib methanesulfonate and methods of preparation thereof.

In WO 99/03854 there are disclosed compositions in the form of tablets and capsules containing imatinib methanesulfonate in the crystalline form beta. The tablets were prepared by direct tabletting, and the capsules were filled with a powder mixture obtained by simple blending all ingredients. Weights of the compositions obtained for imatinib methanesulfonate dose of 100 mg were quite large. For example, to encapsulate a mixture containing 100 mg of imatinib methanesulfonate, a capsule shell size 1 (overall closed length of the capsule: 19.4 mm, maximum diameter: 6.91 mm) was required.

Tablets and capsules containing imatinib methanesulfonate in the crystalline form beta are also disclosed in WO 01 /47507. Similarly to the method described in WO 99/03854, tablets were prepared by direct tabletting, and the capsules were filled with a powder mixture obtained by simple blending all ingredients. For described compositions, tablet and capsule sizes required for accommodation of imatinib methanesulfonate dose of 100 mg were quite significant. For example, for imatinib methanesulfonate dose of 100 mg, capsule of size 1 was required.

Solution of a problem of considerable size of dosage forms for high doses of imatinib was proposed in WO 03/090720, where a tablet containing from 30 to 80% by weight of imatinib based on the total weight of the tablet was disclosed. The tablet was prepared using wet granulation method, preferably using the active ingredient imatinib as methanesulfonate salt in the crystalline form beta.

In WO 2009/042803 there is described a pharmaceutical composition in the form of a tablet, containing 23-29% by weight of imatinib, preferably as methanesulfonate salt. The composition was proposed as a solution to a problem that was noticed for the tablets prepared according to the method described in WO 03/090720. It was stated that although the method described in WO 03/090720 can be used for preparing tablets of relatively small size even with high doses of the active ingredient, the tablets were brittle and showed poor abrasion resistance. The solution proposed in WO 2009/042803 was to increase the content of excipients in the tablet, but at the expense of increasing tablet's weight, and thus its size. For example, the tablet containing imatinib mesylate at a dose of 100 mg had the weight of 412 mg and at a dose of 400 mg the weight of 1648 mg.

In EP1762230A there is described a composition in the form of coated tablets or granulate having high content of imatinib methanesulfonate in the crystalline alpha form (25-80% by weight), produced by a process involving twofold compression. Active ingredient and optionally one of the excipients are compacted or compressed to obtain a granulate, and then the granulate is compressed along with other pharmaceutical additives to form tablets or final granulate.

Prior art publications discussed above show that manufacturing pharmaceutical composition containing high dose of imatinib methanesulfonate in the crystalline alpha form and having relatively small size, without exposure of this active ingredient to a contact with solvents at any stage of manufacturing, gives rise to some problems to the skilled in the art.

Due to the properties of imatinib methanesulfonate in the crystalline alpha form: low bulk density, poor flowability, electrostatic properties, hygroscopicity, metastability of this crystalline form, but favorable solubility profile at the same time, and due to the necessity of administration of relatively high doses of the active ingredient to patients, a more economical manufacturing method of convenient for patients pharmaceutical compositions containing imatinib methanesulfonate form alpha remains a constant challenge for formulation specialists. Particularly important is to ensure the stability of the crystalline alpha form of imatinib methanesulfonate, a proper release profile and the size of a unit dosage form acceptable for patients.

These objectives have been achieved by the present invention, which relates to a solid blend for filling the unit dosage forms, containing granulate of imatinib methanesulfonate in the crystalline alpha form, optionally with the addition of pharmaceutical excipients, with an extragranular lubricant and optionally a glidant. The blend of the invention can be prepared without using any solvents, particularly in the granulation step. The blend of the invention is used for filling the unit dosage forms.

The present invention provides a pharmaceutical composition in the form of a solid blend, the composition consisting of:
a) 99.7 to 96% by weight of granulate of imatinib methanesulfonate, wherein the granulate contains, with respect to the total weight of the granulate, a1) from 81 to 100 % by weight of imatinib methanesulfonate in the crystalline alpha form, and
   a2) from 0 to 19% by weight of one or more pharmaceutical excipients;
b) 0.3 to 3% by weight of an extragranular lubricant, and
c) 0 to 1% by weight of an extragranular glidant.

In a preferred embodiment of the present invention, the granulate a) contains from 82 to 100 % by weight, preferably 82 to 90% by weight, more preferably 84 to 90% by weight and in the most preferred embodiment, 84% by weight of imatinib methanesulfonate, with respect to the total weight of the granulate a).

Apart from imatinib methanesulfonate in the crystalline alpha form, the granulate a) may include one or more pharmaceutically acceptable excipients such as fillers, disintegrants, binders, glidants, lubricants, dissolution enhancers for the active ingredient (solubilizers), flowability enhancers, anti-adhesion agents and/or anti-electrostatic agents.

In particular, the granulate a) in accordance with the present invention contains as one or more pharmaceutical excipients a2) from 0 to 19% by weight of a filler, from 0 to 15% by weight of a disintegrant, from 0 to 19% by weight of a binder and from 0 to 1% by weight of a glidant.

The filler in the granulate a) may be selected from the group consisting of oligosaccharides, such as lactose anhydrous or lactose monohydrate, sucrose, mannitol, polysaccharides, such as microcrystalline cellulose, starch, pregelatinized starch and derivatives thereof, such as carboxymethylcellulose calcium, inorganic excipients, such as calcium carbonate, and mixtures and co-processed mixtures thereof, such as for example silicified microcrystalline cellulose.

Preferably, the granulate a) contains from 5 to 12% by weight, in particular 9% by weight, of the filler.

Preferably, the filler is lactose.

According to the invention, disintegrants in the granulate a) may be selected from the group consisting of carboxymethylcellulose calcium, microcrystalline cellulose, anhydrous colloidal silica, crospovidone, for example crospovidone type A (Polyplasdone XL^{®}), croscarmellose sodium, corn starch, and sodium starch glycolate.

Preferably, the granulate a) contains from 5 to 9% by weight, most preferably 7% by weight of the disintegrant, with respect to the total weight of the granulate.

Preferably, the disintegrant is crospovidone.

Binders in the granulate a) may be selected from the group consisting of carboxymethylcellulose sodium or calcium, hydroxypropylcellulose, hydroxypropyl-methylcellulose, microcrystalline cellulose, hydrogenated vegetable oil, povidone, and pregelatinized corn starch.

According to the invention, the amount of the binder used for the granulation together with the active ingredient is from 0% to 19% by weight, with respect to the total weight of the granulate a). For example, carboxymethylcellulose sodium is used in the amount of from 1 to 6% by weight, and carboxymethylcellulose calcium in the amount of from 5 to 15% by weight.

Glidants in the granulate a) may be selected from the group consisting of anhydrous colloidal silica (Aerosil 200), starch, and talc. Preferably, the glidant is anhydrous colloidal silica in the amount of from 0 to 0.5% by weight, with respect to the total weight of the granulate a).

The granulate a) in the composition of the present invention is blended with the lubricant b), and optionally with the glidant c).

Extragranular lubricant b) may be selected from the group consisting of magnesium stearate, calcium stearate or other salts of stearic acid, sodium stearyl fumarate, hydrogenated vegetable oil, such as hydrogenated castor oil, sodium lauryl sulfate, and PEG. The amount of the extragranular lubricant b) in the composition of the present invention is preferably from 0.3% to 1% by weight, more preferably 0.5% by weight, with respect to the total weight of the solid blend. Advantageously, the extragranular lubricant b) is magnesium stearate.

Extragranular glidant c) may be selected from the group consisting of anhydrous colloidal silica (Aerosil 200), starch, and talc. Preferably, the glidant c) is anhydrous colloidal silica in the amount of from 0 to 0.5% by weight, with respect to the total weight of the solid blend.

The present invention also provides a process for manufacturing the pharmaceutical composition as defined above, consisting of the following steps:
- single dry granulation step of imatinib methanesulfonate in the crystalline alpha form a1), optionally in a mixture with one or more of solid pharmaceutical excipients a2), without the use of any solvents, to obtain a granulate a), and
- the step blending the resulting granulate a) with a lubricant b), and optionally with a glidant c), if present.

As defined above, the process of the invention includes single dry granulation step, which means that granulation of imatinib methanesulfonate in the crystalline alpha form a1), optionally in a mixture with one or more of solid pharmaceutical excipients a2), is carried out only once, without repeating it.

The term "without the use of any solvents" means the granulation is carried out in the absence of any solvent.

In a preferred embodiment of the invention, granulation without using any solvents is carried out by compaction or slugging. Preferably, a roller compactor is used. Compaction pressure of compactor rolls is in the range of 30-130 bar, preferably 80-110 bar, and most preferably 90-100 bar.

Blending the granulate with the lubricant b) and the optional glidant c) may be carried out using mixers conventionally used in pharmaceutical technology.

The composition of the invention after manufacturing thereof is a free-flowing solid blend, and can be used for filling unit dosage forms.

Thus, the process for manufacturing the pharmaceutical composition in the form of solid blend as described above may be followed by a further optional step of filling the solid blend into unit dosage forms.

Thus, another object of the invention is a unit dosage form filled with the pharmaceutical composition of the invention or manufactured using the process of the present invention. The pharmaceutical composition of the invention is contained in such a unit dosage form.

In particular, the unit dosage form is selected from a capsule, a sachet, a stick and an ampoule. Preferred unit dosage form is a hard capsule, for example, gelatin or hydroxypropylmethylcellulose hard capsule. In a most preferred embodiment, the unit dosage form is hard gelatin capsule.

Size of a dosage form, preferably a capsule, depends on the dose of the active ingredient and on the amount of the solid blend containing that dose. The amount of the blend for filling unit dosage form is adjusted so that the content of imatinib methanesulfonate in a unit dosage corresponds to 50, 100, 200, 300, 400 or 600 mg of imatinib free base.

The dosage form of the invention may therefore include imatinib methanesulfonate in the crystalline alpha form in the amount corresponding to 50, 100, 200, 300, 400 or 600 mg of imatinib free base per dosage unit.

For example, the amount of the solid blend of the invention containing imatinib methanesulfonate in the amount corresponding to 100 mg of imatinib free base is suitable for filling capsules of size 3, while the amount of the solid blend of the invention containing imatinib methanesulfonate in the amount corresponding to 400 mg of imatinib free base is suitable for filling capsules of size 00. Capsules of the suitable parameters are commercially available (Capsugel, Qualicaps). In Example 3 capsules Coni-Snap^{®} of size 3 (total length of the capsule: 15.9 mm, maximum diameter: 5.82 mm) of size 00 (total length: 23.3 mm, maximum diameter: 8 , 53 mm) from Capsugel were used.

Filling the unit dosage forms, in particular capsules, with the composition of the invention is a well known process in the pharmaceutical technology. Filling the capsules may be carried out by gravity filling or by using dosators of various types, depending on the type of encapsulation machine.

### Examples

### Example 1. Manufacturing the granulate

Imatinib methanesulfonate (crystalline alpha form) and other components of the granulate were dosed in the amounts as indicated in Table 1 and mixed vigorously for 20 minutes using high shear mixer/granulator Glatt. The resulting mixture was compacted using roller compactor Alexanderwerk, while applying compaction pressure of compactor rolls of 90 bar and screen of 0.63 mm.

**Table 1.**

| Granulate | Imatinib methanesulfonate | Lactose monohydrate 200 M* (filler) | Crospovidone Polyplasdone XL* (disintegrant) | Aerosil* (glidant) |
|---|---|---|---|---|
| Granulate 1 | 1792.05 g (100%) | - | - | - |
| Granulate 2 | 1792.05 g (90%) | 99.56 g (5%) | 99.56 g (5%) | - |
| Granulate 3 | 1792.05 g (84%) | 192.01 g (9%) | 149.34 g (7%) | - |
| Granulate 4 | 1792.05 g (82%) | 218.54 g (10%) | 174.83 g (8%) | - |
| Granulate 5 | 1792.05 g (81%) | 265.49 g (12%) | 143.81 g (6.5%) | 11.06 g (0.5%) |
| Granulate 6 | 1792.05 g (86%) | 145.87 g (7%) | 135.45 g (6.5%) | 10.419 g (0.5%) |

| | | | | |
|---|---|---|---|---|
| * amounts of components are expressed in grams, and % by weight of the component, with respect to the total weight of the granulate, is given in brackets | | | | |

### Example 2. Manufacturing the solid blend

The granulate of Example 1, the lubricant magnesium stearate and optionally the glidant Aerosil in the amounts as given in Table 2, were blended together in the mixer Pharmatech (mixing time: 5 minutes).

**Table 2.**

| Blend | Granulate* | Magnesium stearate (lubricant)* | Aerosil* (glidant) |
|---|---|---|---|
| Blend 1 | Granulate 1: 1500 g (99%) | 7.58 g (0.5%) | 7,58 g (0.5%) |
| Blend 2 | Granulate 2: 1850 g (99,5%) | 9.30 g (0.5%) | - |
| Blend 3 | Granulate 3: 1900 g (99,5%) | 9.55 g (0.5%) | - |
| Blend 4 | Granulate 4: 1900 g (99%) | 9.56 g (0.5%) | 9,56 g (0.5%) |
| Blend 5 | Granulate 5: 2000 g g (99,5%) | 10.05 g (0.5%) | - |
| Blend 6 | Granulate :6 1900 g (99,5%) | 9.55 g (0.5%) | - |

| | | | |
|---|---|---|---|
| * amounts of components are expressed in grams, and % by weight of the component, based on the total weight of the blend, is given in brackets | | | |

### Example 3. Filling the capsules

The blend of Example 2 was used for filling hard gelatine capsules of size adjusted to the required dose of the active ingredient. Encapsulation was performed using encapsulation machine MG Compact by MG2.

**Table 3.**

| | Capsule 100 mg* | Capsule 400 mg* |
|---|---|---|
| Capsule size | 3 | 00 |
| Empty capsule weight [mg] | 48 | 118 |
| Blend 1 [mg/ unit dosage] | 120.68 | 482.71 |
| Blend 2 [mg/ unit dosage] | 133.41 | 533.65 |
| Blend 3 [mg/ unit dosage] | 142.94 | 571.76 |
| Blend 4 [mg/ unit dosage] | 147.16 | 588.65 |
| Blend 5 [mg/ unit dosage] | 148.23 | 592.94 |
| Blend 6 [mg/ unit dosage] | 139.62 | 558.47 |

| | | |
|---|---|---|
| * weight of 100 mg and 400 mg corresponds to the weight of imatinib free base in the unit dosage and is equivalent to 119,47 mg and 477,88 mg of imatinib methanesulfonate, respectively | | |

Stability testing of the capsules filled with the solid blend of the invention has shown that imatinib methanesulfonate in the crystalline alpha form contained therein is stable under both normal and accelerated conditions. Composition of the invention shows a suitable release profile and favorable technological properties, such as suitable flowability. Twofold compression in order to provide advantageous properties of the final composition, as described in EP 1762230A, proved to be unnecessary, thus the manufacturing process of a pharmaceutical composition is more simple and more economical.

Moreover, when performing experiments it was observed that subjecting a mixture containing imatinib methanesulfonate crystalline alpha form to repeated compactions may increase the risk of partial conversion of this metastable form into a thermodynamically more stable form. Similar risks would be also bound with the use of solvents during the production of a composition.

Importantly, influence of both those detrimental factors was eliminated in the composition and the process of the present invention. Reducing the amount of excipients in the composition allowed also to obtain unit dosage forms having size acceptable to patients, even at high doses of the active ingredient.

## Claims

1. A pharmaceutical composition of imatinib methanesulfonate, which composition is in the form of a solid blend and consists of:
a) from 99,7 to 96% by weight of a granulate of imatinib methanesulfonate, wherein the granulate contains, with respect to the total weight of the granulate
a1) from 81 to 100% by weight of imatinib methanesulfonate in the crystalline form alpha, and
a2) from 0 to 19% by weight of one or more pharmaceutical excipients;
b) from 0,3 to 3% by weight of an extragranular lubricant; and
c) from 0 to 1% by weight of an extragranular glidant.

2. The pharmaceutical composition according to claim 1, wherein the granulate a) contains 84 to 90% by weight of a1) imatinib methanesulfonate.

3. The pharmaceutical composition according to claim 1 or 2, wherein the granulate a) contains as one or more pharmaceutical excipients a2) from 0 to 19% by weight of a filler, from 0 to 15% by weight of a disintegrant, from 0 to 19% by weight of a binder and from 0 to 1% by weight of a glidant.

4. The pharmaceutical composition according to claim 1, 2 or 3, wherein the granulate a) contains from 5 to 12% by weight, especially 9% by weight of the filler.

5. The pharmaceutical composition according to one of claims 1 to 4, wherein the granulate a) contains from 5 to 9% by weight, especially 7% by weight of the disintegrant.

6. The pharmaceutical composition according to one of claims 1 to 5, wherein the granulate a) contains 0 to 0,5% by weight of the glidant.

7. The pharmaceutical composition according to one of claims 1 to 6, wherein it contains from 0,3 do 1% by weight, especially 0.5% by weight of the extragranular lubricant b).

8. The pharmaceutical composition according to one of claims 1 to 7, wherein it contains from 0 to 0.5% by weight of the glidant c).

9. A unit dosage form filled with the pharmaceutical composition as defined in any one of claims 1 to 8.

10. The unit dosage form according to claim 9, selected from the group consisting of a capsule, a sachet, a stick and an ampoule.

11. The unit dosage form according to claim 10, wherein the capsule is a hard gelatin capsule.

12. The unit dosage form according to claim 9, 10 or 11, wherein the amount of imatinib methanesulfonate per unit dosage form corresponds to 50, 100, 200, 300, 400 or 600 mg of imatinib free base.

13. A process for manufacturing the composition in the form of a solid blend as defined in any one of claims 1 to 8 or the unit dosage form as defined in any one of claims 9-12, consisting of the following steps:
- single dry granulation step of imatinib methanesulfonate a1), optionally in a mixture with one or more pharmaceutical excipients a2), without using any solvents, to obtain the granulate a), and
- the step of blending the resulting granulate a) with the lubricant b) and with the glidant c), if present, to obtain the solid blend,
and optionally, to obtain the unit dosage form
- the step of filling the solid blend into a unit dosage form.

14. The process according to claim 13, wherein dry granulation is carried out by compaction or slugging.

15. The process according to claim 14, wherein compaction is carried out using roller compactor.

16. The process according to claim 15, wherein the compaction pressure of compactor rolls is in the range of 90 to 100 bar.
